# EUROPEAN PATENT APPLICATION

(11) **EP 1 318 202 A1**
(43) Date of publication of application: **11.06.2003**
(21) Application number: 01965574.5
(22) Date of filing: 12.09.2001
(51) Int. Cl.: C12Q 1/48, G01N 33/53

(54) **METHOD OF DIAGNOSING OSTEOPOROSIS AND/OR ESTIMATING RISK OF OSTEOPOROSIS FRACTURE**

(30) Priority: 14.09.2000 JP 2000279261
(71) Applicant: Applied Cell Biotechnologies, Inc., Tsukuba-shi Ibaraki 305-0028 (JP)
(72) Inventor: NIIDA, Shunpei, Hiroshima-shi Hiroshima 734-0004 (JP); SANADA, Mitsuhiro, Hiroshima 734-0015 (JP); ISHIZUKA, Yasuyuki, Kawasaki-shi Kanagawa 215-0015 (JP)
(74) Representative: Trösch, Hans-Ludwig
(86) International application number: JP0107897
(87) International publication number: WO02022863

(57) **Abstract**

Objects of the invention are to provide a new turnover marker and on the basis of this, to provide a method capable of diagnosis of osteoporosis and/or prediction of osteoporotic fracture risk quickly, conveniently, and accurately. To solve the problems, urine γ-GTP is measured.

## Description

### Technical Field

The present invention relates to quickly-, conveniently- and accurately-operable method for diagnosis of osteoporosis and/ or presumption of osteoporotic fracture risk.

### Background of the Invention

Osteoporosis, that is characterized in that bone density decreases keeping normal bone composition, is a disease frequently occurring in a woman and a girl. Particularly, the woman of age 40s or more shows postmenopausal osteoporosis related to menopause in a high frequency. After 70s, senile osteoporosis occurs regardless of sex.

During decrease in bone density shows a low degree, no prominent clinical symptom appears. However, the degree of decrease becomes higher, fracture and bone deformation become easy to occur. A spine is easy to occur fracture and bone deformation and such symptoms as compressed fracture of a body of vertebra and lumbago caused by spinal deformation appear.

Moreover, fracture of a femoral neck and a pubis may damage a motor function of a patient to cause serious effect on a life thereafter.

At present, osteoporosis is diagnosed by means of such physical technique as X-ray photography. However, This technique requires a large-scale diagnostic apparatus and has defects in that a safety problem arises through using X-rays and diagnosis can be carried out only after onset, where bone density decreases prominently.

Consequently, through early detection and diagnosis of various types of osteoporosis such as postmenopausal osteoporosis and senile osteoporosis to predict a fracture risk, is desired establishing quick, convenient, and accurate method to make effective treatment possible.

In recent years, a rapidly developed method for diagnosing osteoporosis is to detect a factor relating to bone metabolism from blood and urine followed by measuring this as a marker of bone metabolism. The bone metabolism marker is mainly classified into an turnover marker and an osteogenesis marker. Among them, pyridinoline (Pyr) being a decomposition product of an type I collagen-crosslinking component being a main component of a matrix of bones, deoxypyridinoline (D-Pyr,) a telopeptide fragment of a C terminal of the type 1 collagen (CTx,) the telopeptide of a C terminal of the type I collagen (ICTP,) the telopeptide fragment of an N terminal of the type I collagen (NTx,) and tartaric acid-resistant acid phosphatase (TRAP) produced by an osteoclast are known as useful as the turnover marker. However, in consideration of specificity and sensitivity, a more excellent turnover marker is being searched.

In such situation, a purpose of the present invention is to provide a method for quickly-, conveniently- and accurately-operable method for diagnosis of osteoporosis and/ or presumption of osteoporotic fracture risk on the basis of providing a new turnover marker.

### Disclosure of the Invention

The present inventors conducted various studies in consideration of the above described points. As a result, it was found that urine γ-glutamyl transpeptidase (hereafter γ-GTP) functions as the turnover marker, that more γ-GTP makes bone density low to develop osteoporosis, and that a trend of decrease in the turnover marker by administration of therapeutic agent for osteoporosis coincides with the trend of recovery (increase) of bone density.

Also their finding is that using this relation makes diagnosis of osteoporosis and/ or and the prediction of osteoporotic fracture risk quick, convenient, and accurate.

γ-GTP is a transfer enzyme widely known as a marker reflecting specifically hepatic and cholangial diseases. Japanese Published Unexamined Patent Application No. 1998-87507 has disclosed that γ-GTP has an osteoclast differentiation-enhancing activity. On the contrary, it is a fact unexpected from the description of the above described publication that a specific function of urine γ-GTP as the turnover marker was found.

The present invention was created on the basis of the above described findings. The method, according to the present invention, for diagnosis of osteoporosis and/ or prediction of osteoporotic fracture risk is characterized in that urine γ-GTP is measured according to the description of claim 1.

The method according to the claim 2 is characterized in that γ -GTP is measured by measuring a γ-GTP activity through knowing a degree of coloration by adding a substrate for measurement of the γ-GTP activity to urine in the method according to the claim 1.

A monitoring method, according to the present invention, for bone density is characterized in that urine γ-GTP is measured according to the claim 3.

The present invention is characterized in that urine γ-GTP is used as a turnover marker according to the claim 4.

A reagent according to the present invention is characterized, according to the claim 5, in that the reagent contains at least the substrate for measurement of the γ-GTP activity and is used for diagnosis of osteoporosis and/ or prediction of osteoporotic fracture risk.

### Brief Description of the Drawings

Fig. 1 is a graph showing a correlation of urine γ-GTP with Urine NTx before a start of a therapy.
Fig. 2 is a graph showing the correlation of both measures at 3 months after the therapy.
Fig. 3 is a graph showing the correlation of both measures at 6 months after the therapy.
Fig. 4 is a graph showing the correlation of both measures at 12 months after the therapy.
Fig. 5 is a graph showing trends of urine γ-GTP, Urine NTx, and bone density during the therapy.

### The Best Mode for Carrying Out the Claimed Invention

The Embodiment of the present invention will be described below. The method, according to the present invention, for diagnosis of osteoporosis and/ or prediction of osteoporotic fracture risk is characterized by measuring urine γ-GTP.

The method for measuring urine γ-GTP is not specially restricted. Applicable methods may be exemplified by enzyme immunoassay (EIA) using an antibody specific to γ-GTP, radio immunoassay (RIA,) and a quantifying method employing high performance liquid chromatography (HPLC.) Particularly, a preferable method (enzyme method) is carried out by adding the substrate for measurement of the γ-GTP activity to urine and measuring the degree of coloration as the γ-GTP activity. The enzyme method is advantageous in that no pretreatment is required for a sample to allow presenting a result in a short time resulting in a rapid and convenient measurement. On the other hand, various kinds of reagents used for measurement in this method are commercialized as diagnostic reagents for hepatic and cholangial diseases. In the present invention, measurement is carried out by applying a same method as an operation method (a direction and a dose) for diagnosis of hepatic and cholangial diseases, using the commercialized reagents for measurement. A value of γ-GTP measured is corrected using a urine creatinine value if necessary.

The substrate used for measurement of the γ-GTP activity is exemplified by following various synthesized substrates contained in the above described commercialized reagents for diagnosis: L- γ-glutamyl-p-nitroanilid, L-γ-glutamyl-3-carboxy-4-nitroanilid, L- γ-glutamyl-3-carboxy-4-hydroxyanilid, L-γ-glutamyl-p-N-ethyl-N- hydroxyethyl aminoanilid, L- γ -glutamyl-3,5-dibromo-4-hydroxy anilid, L-γ-glutamyl-α-naphthyl amine, and L-γ-glutamyl-3-carboxy-1,4-phenylenediamine. Normally, as an acceptor substance of an L-glutamyl group, is added glycylglycine.

The γ-GTP activity is measured using the degree of coloration of a coloring matter produced by liberation from the substrate used for measurement of the γ-GTP activity and the coloring matter derived from a matter produced by liberation from the substrate. Preferably, more precise measurement is carried out by colorimetry. Colorimetry can be performed by the same method as that for colorimetry in diagnosis of hepatic and cholangial diseases. By using various autoanalyzers makes processing of a large quantity of test samples for a short time possible.

For convenient measurement, visual measurement may be applied. In this case, urine as the test sample requires coloration other than yellow. The substrates capable of appearing coloration other than yellow are exemplified by L- γ -glutamyl-3,5-dibromo-4-hydroxy anilid (3,5-dibromo-4-hydroxy anilid (DBHA) is liberated and produced and a green pigment is produced by oxidative condensation reaction of DBHA with N-ethyl-N-(3-methylphenyl)-N'-succinyl ethylenediamine through an action of phenyl monooxygenase.)

According to the measurement of urine γ-GTP by the above described method, γ-GTP works as the turnover marker and thus, the more the quantity of γ-GTP the lower the bone density meaning development of osteoporosis. On the other hand, decrease in γ-GTP corresponds to recovery (increase) of bone density meaning an improvement of the symptom. Therefore, using this correlation makes diagnosis of osteoporosis and/ or and the prediction of osteoporotic fracture risk quick, convenient, and accurate. In addition, monitoring of bone density by a plurality of measurement frequencies of urine γ-GTP of individual patients allows easy operation of choosing an osteoporosis remedy and determining an effect thereof.

The present invention will be specifically described as follows with reference to examples. The present invention is, however, in no way restricted to these examples.

### <Method>

35 objective menopausal women patients, who have no hepatic and cholangial diseases and were not subjected to a therapy for osteoporosis, were subjected to measurement of serum γ-GTP and urine γ-GTP with informed consent of them (all measurements were carried out by applying the enzyme method using Merck Liquid γ-GT J made by Kanto Kagaku Corporation as a reagent for the measurement and by operating the type 7170 autoanalyzer made by Hitachi Corporation after the operation manual.)

A urine telopeptide fragment of the N terminal of the type I collagen (NTx) and serum alkali phosphatase (ALP) were measured as the turnover marker of a control group and the osteogenesis marker of the control group, respectively (measurements of the former and the latter were conducted by applying the ELISA method using the type L Wako ALP-J made by Wako Pure Chemicals, Corporation as the reagent for the measurement and the enzyme method using "Osteomark" made by Mochida Pharmaceutical Co. Ltd. as the reagent for the measurement, respectively.)

Bone density of a lumbar spine and a femoral neck was measured by using DPX made by Lunar Co.

Starting from a point where the above measurement was carried out, independent administration of a 0.625 mg/ day of conjugated estrogen or combined administration of this with 2.5 mg/ day of medroxyprogesterone acetate was carried out as a treatment by hormone-replacement therapy for individual patients for 2 months. After this treatment, urine and serum γ-GTP, urine NTx, and serum ALP were measured 3, 6, and 12 months after the start of the treatment. Bone density was measured 6 and 12 months after the start of the treatment.

### <Result>

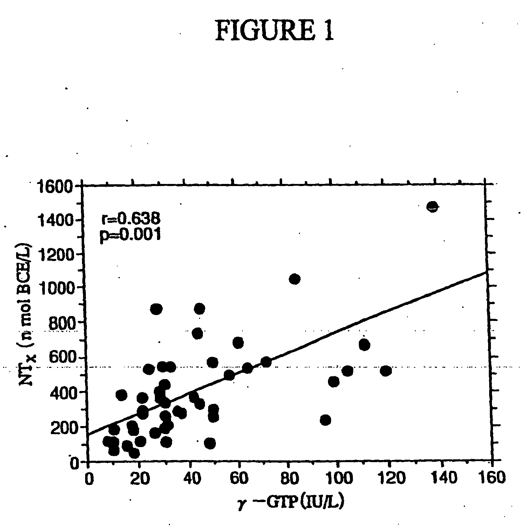

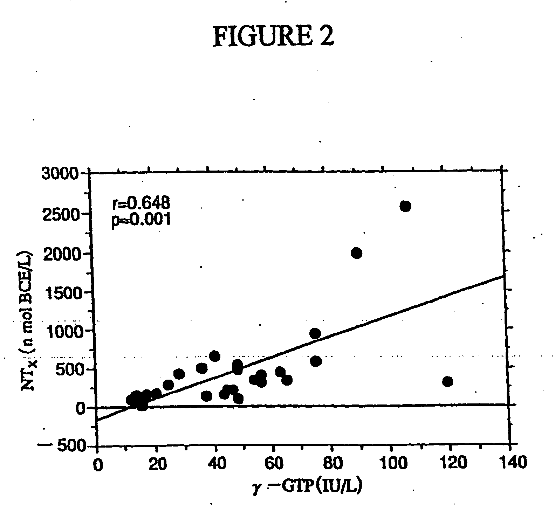

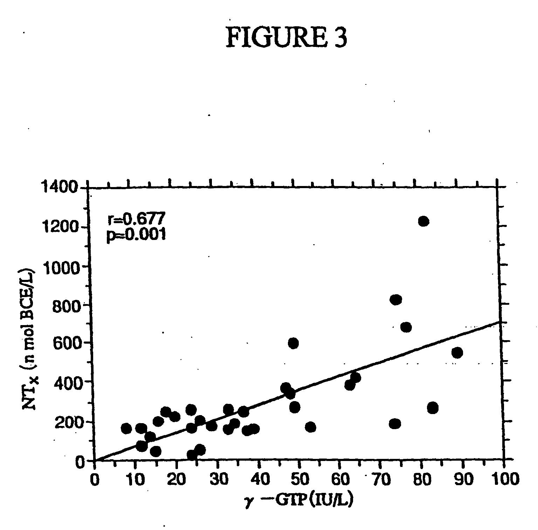

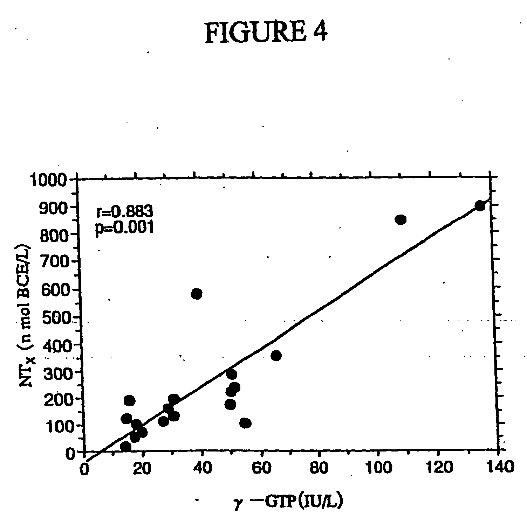

During the treatment by hormone-replacement therapy, urine γ-GTP showed significant positive correlation with urine NTx (refer to Fig. 1 to Fig.4.) Either of the measures showed similar trends resulting in reduction of 21.4% and 37.7% in averages of urine γ-GTP and urine NTx, respectively, 12 months after the treatment.

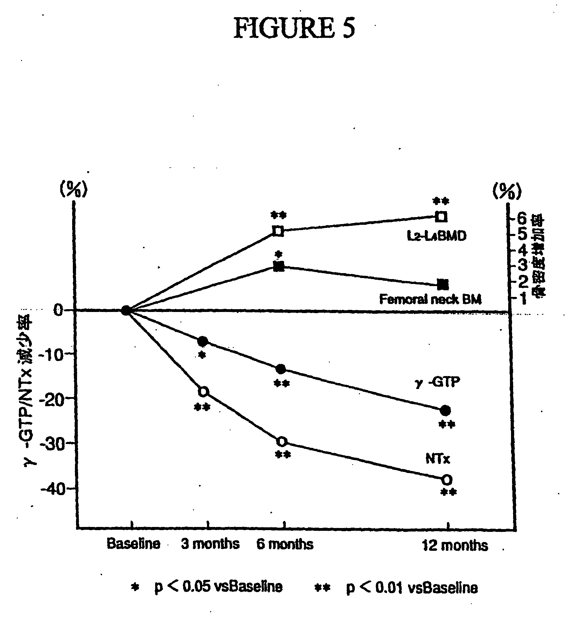

Regarding bone density, bone density of the lumbar spine (L2-L4 BMD) and the femoral neck (femoral neck BMD) increased for 6.0% and 1.7% in averages, respectively, 12 months after the treatment (refer to Fig. 5.) Serum ALP decreased fro 18.5% in average 12 months after the treatment. Serum γ -GTP showed no any significant variation during the treatment.

From the above described result, it was found that the more the urine γ-GTP the lower and the bone density, that decrease in γ-GTP caused by hormone-replacement therapy coincides with recovery (increase) of bone density, and that urine γ-GTP shows the similar trend to that of urine NTx of which usefulness as the turnover marker has been recognized at present. Therefore, it can be said that urine γ-GTP works as the turnover marker.

As described above, γ-GTP has the osteoclast differentiation-enhancing activity and hence, urine γ-GTP is useful as the marker having a property that any existing turnover marker has not.

### Industrial Applicability

According to the present invention, Urine γ-GTP is provided as a new turnover marker. Measuring this makes diagnosis of osteoporosis and/ or and the prediction of osteoporotic fracture risk quick, convenient, and accurate.

## Claims

1. A method for diagnosis of osteoporosis and/ or prediction of osteoporotic fracture risk by means of measuring urine γ-glutamyl transpeptidase (γ-GTP.)

2. The method according to claim 1, wherein γ-GTP is measured by measuring a degree of coloration as a γ-GTP activity through adding a substrate for measurement of the γ-GTP activity to urine.

3. A method for monitoring bone density by measuring urine γ-GTP.

4. Use of urine γ-GTP as an turnover marker.

5. A reagent that contains at least the substrate for measurement of the γ-GTP activity and is used for diagnosis of osteoporosis and/ or prediction of osteoporotic fracture risk.
